# EUROPÄISCHE PATENTSCHRIFT

(11) **EP 3 585 455 B1**
(45) Veröffentlichungstag und Bekanntmachung des Hinweises auf die Patenterteilung: **22.09.2021**
(21) Anmeldenummer: 18707322.6
(22) Anmeldetag: 21.02.2018
(51) Int. Cl.: A61M 1/16, A61M 39/22

(54) **EXTRAKORPORALE BLUTBEHANDLUNGSVORRICHTUNG UND VERFAHREN ZUM BETREIBEN EINER EXTRAKORPORALEN BLUTBEHANDLUNGSVORRICHTUNG**
EXTRACORPOREAL BLOOD TREATMENT DEVICE AND METHOD FOR OPERATING AN EXTRACORPOREAL BLOOD TREATMENT DEVICE
DISPOSITIF DE TRAITEMENT EXTRACORPOREL DU SANG ET PROCÉDÉ POUR FAIRE FONCTIONNER UN DISPOSITIF DE TRAITEMENT EXTRACORPOREL DU SANG

(30) Priorität: 23.02.2017 DE 102017001770
(43) Veröffentlichungstag der Anmeldung: 01.01.2020
(73) Patentinhaber: Fresenius Medical Care Deutschland GmbH, 61352 Bad Homburg v.d.H (DE)
(72) Erfinder: IRRGANG, Tobias, 97633 Aubstadt (DE)
(74) Vertreter: Oppermann, Frank
(86) Internationale Anmeldenummer: PCT/EP2018/054197
(87) Internationale Veröffentlichungsnummer: WO 2018/153881

(56) Entgegenhaltungen:
- US-A- 4 695 385
- US-A- 5 948 247
- US-A- 6 153 102
- US-A1- 2012 308 431
- US-B1- 6 607 697

## Beschreibung

Die Erfindung betrifft eine extrakorporale Blutbehandlungsvorrichtung, die ein Hydrauliksystem aufweist, das mehrere Strömungsstrecken umfasst, wobei die Blutbehandlungsvorrichtung eine Steuereinheit aufweist, die einen Betriebsmodus für eine Desinfektion des Hydrauliksystems mit einer Flüssigkeit vorsieht, die über eine der Strömungsstrecken zuführbar ist. Darüber hinaus betrifft die Erfindung ein Verfahren zum Betreiben einer extrakorporalen Blutbehandlungsvorrichtung mit einem mehrere Strömungsstrecken umfassenden Hydrauliksystem.

Bei der Dialyse durchströmt das zu behandelnde Blut in einem extrakorporalen Blutkreislauf die Blutkammer eines durch eine semipermeable Membran in die Blutkammer und eine Dialysatkammer unterteilten Dialysators, während Dialysat die Dialysatkammer des Dialysators durchströmt. Der extrakorporale Blutkreislauf umfasst eine Blutzuführleitung, die zu der Blutkammer führt, und eine Blutabführleitung, die von der Blutkammer abgeht. Das Hydrauliksystem der extrakorporalen Blutbehandlungsvorrichtung umfasst mehrere Baugruppen zur Aufbereitung und Bilanzierung des Dialysats, die wiederum über mehrere Komponenten verfügen können. Die einzelnen Komponenten des Hydrauliksystems stehen über mehrere Strömungsstrecken in Fluidverbindung. Die einzelnen Strömungsstrecken können eine oder mehrere Leitungen aufweisen. Dem Dialysator wird beispielsweise frisches Dialysat über eine Dialysatzuführleitung zugeführt und gebrauchtes Dialysat wird über eine Dialysatabführleitung von dem Dialysator abgeführt. Zum Fördern der Flüssigkeiten sind Pumpen vorgesehen sein. Die Steuerung der einzelnen Komponenten der Blutbehandlungsvorrichtung erfolgt mit einer Steuereinheit.

Das Dialysat kann in der Dialysemaschine aus Permeat (Reinwasser) und einem oder mehreren Konzentraten hergestellt werden. Für die Zuführung des Permeats weisen die Dialysemaschinen einen Wasseranschluss auf.

Die US 4,695,385 A beschreibt eine extrakorporale Blutbehandlungsvorrichtung, die ein Hydrauliksystem aufweist, das mehrere Strömungsstrecken umfasst. Die Blutbehandlungsvorrichtung sieht einen Betriebsmodus für eine Desinfektion des Hydrauliksystems mit einer Desinfektionsflüssigkeit vor. In dem Desinfektionsmodus strömt Desinfektionsflüssigkeit durch die Schlauchleitungen des Schlauchleitungssystems in die bzw. aus der Dialysatkammer und in die bzw. aus der Blutkammer des Dialysators. Das Hydrauliksystem verfügt über eine Ventileinrichtung mit Einlässen und Auslässen, an denen die Schlauchleitungen angeschlossen sind. Die Ventileinrichtung sieht eine Schaltstellung für den Behandlungsmodus und eine Schaltstellung für den Desinfektionsmodus vor.

Für die Desinfektion des Hydrauliksystems der Blutbehandlungsvorrichtungen gewinnt die Heißdesinfektion an Bedeutung, um den Einsatz von Chemikalien zu verringern. Bei der Heißdesinfektion wird dem Hydrauliksystem auf eine Temperatur von größer als 80°C erwärmtes Reinwasser (Permeat) zugeführt. Während der Heißdesinfektion zirkuliert das erhitzte Permeat in den Strömungsstrecken des Hydrauliksystems. Für eine ausreichende Desinfektion ist entscheidend, dass die vorgegebene Temperatur des Permeats in sämtlichen Strömungstrecken des Hydrauliksystems nicht unterschritten wird.

Blutbehandlungsvorrichtungen mit einer Einrichtung zur Heißdesinfektion sind aus der US 6 153 102 und der EP 0 208 090 A1 bekannt.

Das erhitzte Permeat wird über eine der Strömungsstrecken des Hydrauliksystems zugeführt. Dann verteilt sich das Permeat in den anderen Strömungsstrecken. In der Praxis hat sich gezeigt, dass bei einer zentralen Zuführung des erhitzten Permeats über einen der Strömungswege das Permeat in einzelnen Strömungsstrecken, die von der zentralen Zuführung weiter entfernt sind, auf eine Temperatur abgekühlt ist, die unterhalb der Temperatur liegt, die das Permeat in einzelnen Strömungsstrecken hat, die von der zentralen Zuführung nicht soweit entfernt sind. Die Abkühlung der Desinfektionsflüssigkeit kann zwar durch längere Desinfektionszeiten oder Chemikalienzugabe kompensiert werden. Längere Desinfektionszeiten führen aber zu einem höheren Energieverbrauch und eine Chemikalienzugabe zu einem höheren Chemikalienverbrauch.

Der Erfindung liegt die Aufgabe zugrunde, eine extrakorporale Blutbehandlungsvorrichtung zu schaffen, bei der während einer Heißdesinfektion auch bei kürzeren Desinfektionszeiten und/oder ohne oder nur geringe Chemikalienzugabe nicht die Gefahr der Unterschreitung der für die Heißdesinfektion erforderlichen Temperatur besteht.

Darüber hinaus liegt der Erfindung die Aufgabe zugrunde, ein Verfahren zum Betreiben einer extrakorporalen Blutbehandlungsvorrichtung anzugeben, das auch bei kürzeren Desinfektionszeiten und/oder ohne oder nur geringe Chemikalienzugabe eine Heißdesinfektion ohne die Gefahr des Unterschreitens der für die Heißdesinfektion erforderlichen Temperatur erlaubt.

Eine weitere Aufgabe der Erfindung liegt darin, die Desinfektionszeiten zu verkürzen und/oder den Energieverbrauch und/oder den Chemikalienverbrauch zu verringern.

Die Lösung dieser Aufgaben erfolgt erfindungsgemäß mit den Merkmalen der unabhängigen Patentansprüche. Die abhängigen Ansprüche betreffen vorteilhafte Ausführungsformen der Erfindung.

Die erfindungsgemäße Blutbehandlungsvorrichtung zeichnet sich dadurch aus, dass das Hydrauliksystem eine mehrere Schaltstellungen vorsehende Ventileinrichtung aufweist. Die Ventileinrichtung weist einen Einlass für die Flüssigkeit zur Desinfektion des Hydrauliksystems und mehrere Auslässe auf. In einer Schaltstellung ist der Einlass mit mehreren Auslässen verbunden. In einer anderen Schaltstellung kann der Einlass mit keinem der Auslässe oder mit einem der Auslässe verbunden sein.

In diesem Zusammenhang wird unter mehreren Auslässen eine Mehrzahl von Auslässen verstanden, die mindestens zwei Auslässe umfasst. Beispielsweise kann die Ventileinrichtung als ein Mehrwegeventil mit einem Einlass und zwei oder drei Auslässen ausgebildet sein. Die Ventileinrichtung kann auch mehr als einen Einlass umfassen, wenn der Ventileinrichtung über mehrere Leitungen eine Flüssigkeit zugeführt werden soll. Das Hydrauliksystem kann auch mehrere Ventileinrichtungen umfassen.

Der Einlass der Ventileinrichtung steht mit der Strömungsstrecke in Fluidverbindung, über die Flüssigkeit zur Desinfektion des Hydrauliksystems zuführbar ist, und die Auslässe der Ventileinrichtung stehen jeweils mit einer anderen Strömungsstrecke des Hydrauliksystems in Fluidverbindung, so dass in der Schaltstellung, in der der Einlass mit mehreren Auslässen verbunden ist, den einzelnen Strömungsstrecken gleichzeitig Flüssigkeit zugeführt werden kann. Beispielsweise können von den Auslässen der Ventileinrichtung Bypass-Leitungen zu anderen Strömungsstrecken führen.

Die gleichzeitige Zuführung der Flüssigkeit an unterschiedlichen Stellen des Hydrauliksystems führt zu geringeren Wegstrecken. Längere Wegstrecken werden somit vermieden, auf denen die Flüssigkeit abkühlen könnte. Folglich ist sichergestellt, dass die Flüssigkeit in sämtlichen Strömungsstrecken die für die Heißdesinfektion erforderliche Temperatur hat. Eine Ventileinrichtung, die zwei Auslässe aufweist, erlaubt die gleichzeitige Versorgung von zwei Strömungswegen mit heißer Flüssigkeit. Wenn die Ventileinrichtung beispielsweise drei Auslässe aufweist, können drei Strömungswege gleichzeitig mit heißer Flüssigkeit versorgt werden.

Die Ventileinrichtung, mit der die Flüssigkeitsströmung in mehrere Strömungstrecken gesteuert werden kann, d.h. freigegeben oder gesperrt werden kann, erlaubt eine Heißdesinfektion auch in denjenigen Strömungstrecken des Hydrauliksystems, die ansonsten nur schlecht oder möglicherweise überhaupt nicht mit heißer Flüssigkeit versorgt werden könnten.

Eine bevorzugte Ausführungsform sieht vor, dass die Blutbehandlungsvorrichtung für die Heißdesinfektion eine Heißdesinfektionseinrichtung mit einer Heizeinheit zum Erhitzen einer Flüssigkeit aufweist, so dass die erhitzte Flüssigkeit in der Blutbehandlungsvorrichtung bereitgestellt werden kann. Die Strömungsstrecke, über die die Flüssigkeit zur Desinfektion des Hydrauliksystems zuführbar ist, steht dann mit der Heißdesinfektionseinrichtung in Fluidverbindung. Für die Zufuhr einer Flüssigkeit, insbesondere Permeat, weist die Blutbehandlungsvorrichtung vorzugsweise einen Anschluss auf, mit dem die Strömungsstrecke, über die die Flüssigkeit zur Desinfektion des Hydrauliksystems zuführbar ist, in Fluidverbindung steht. Die Strömungsstrecke für die Zufuhr der Flüssigkeit kann also die von einem Wasseranschluss über die Heißdesinfektionseinrichtung bis zu der Ventileinrichtung führenden Leitungen umfassen. Die Ventileinrichtung ist im Hydrauliksystem vorzugsweise in enger räumlicher Nähe zu der Heißdesinfektionseinrichtung angeordnet, so dass die Leitungsabschnitte kurz gehalten werden können. Die für die Heißdesinfektion verwendete Flüssigkeit kann auch zur Herstellung der Dialysierflüssigkeit verwendet werden.

Eine weitere bevorzugte Ausführungsform sieht vor, dass das Hydrauliksystem eine zu einem Dialysator führende Dialysatzuführleitung und eine von dem Dialysator abgehende Dialysatabführleitung aufweist, wobei der Einlass der Ventileinrichtung mit der zu dem Dialysator führenden Dialysatzuführleitung in Fluidverbindung steht. Diese Fluidverbindung kann dadurch hergestellt werden, dass die Ventileinrichtung in die Dialysatzuführleitung geschaltet ist oder der Einlass der Ventileinrichtung an eine Leitung angeschlossen ist, die von der Dialysatzufuhrleitung abzweigt.

Der Dialysator weist vorzugsweise einen Einlass zum Zuführen von Dialysat und einen Auslass zum Abführen von Dialysat auf, wobei die Dialysatzuführleitung ein Anschlussstück zum Anschluss an den Einlass des Dialysators und die Dialysatabführleitung ein Anschlusstück zum Anschluss an den Auslass des Dialysators aufweist, so dass der Dialysator für die Desinfektion von dem Hydrauliksystem abtrennbar ist. Die Dialysatzuführleitung endet somit an dem einlassseitigen Anschlussstück.

Das Hydrauliksystem weist eine zu einem Dialysator führende Dialysatzuführleitung und eine von dem Dialysator abgehende Dialysatabführleitung auf, wobei einer der Auslässe der Ventileinrichtung über eine Bypass-Leitung mit der Dialysatzuführleitung verbunden ist. In der Praxis hat sich gezeigt, dass sich die für die Heißdesinfektion verwendete Flüssigkeit in der Dialysatzuführleitung aufgrund der relativ großen Leitungslängen relativ stark abgekühlt hat. Dies wird aber durch die direkte Zufuhr von heißer Flüssigkeit über die Bypass-Leitung in die Dialysatzuführleitung wirksam verhindert. Der Dialysator ist während der Heißdesinfektion von der Dialysatzuführleitung und der Dialysatabführleitung abgetrennt und die Zuführ- und Abführleitungen sind beispielsweise über eine Bypass-Leitung oder ein anderes Verbindungsstück miteinander verbunden. Die direkte Zufuhr von heißer Flüssigkeit in die Dialysatzuftihrleitung kann beispielsweise an einer Stelle stromab eines in die Dialysatzuführleitung geschalteten Filters zur Erhöhung des Reinheitsgrades des Dialysats erfolgen.

Das Hydrauliksystem verfügt über einen oder mehrere Filter zur Erhöhung des Reinheitsgrades des Dialysats, die jeweils in einer der Strömungsstrecken des Hydrauliksystems angeordnet sind, wobei einer der Auslässe der Ventileinrichtung über eine Bypass-Leitung mit einer Strömungsstrecke verbunden ist, in der der Filter zur Erhöhung des Reinheitsgrades des Dialysats angeordnet ist bzw. die mit dem Filter in Fluidverbindung steht, vorzugsweise zu dem Filter führt. Folglich kann auch diesem Teil des Hydrauliksystems heiße Flüssigkeit direkt zugeführt werden.

Die direkte Zufuhr von heißer Flüssigkeit zu den obigen Teilen des Hydrauliksystems ist aber nur als ein Beispiel zu verstehen. Eine direkte Zufuhr ist mit der Ventileinrichtung zu sämtlichen Teilen des Hydrauliksystems möglich, die sich ansonsten nur schlecht erreichen ließen bzw. bei denen die Gefahr besteht, dass die Flüssigkeit aufgrund zu großer Leitungslängen abkühlt.

Bei einer bevorzugten Ausführungsform ist die Ventileinrichtung derart ausgebildet, dass diese von der Steuereinheit betätigbar ist. Die Ventileinrichtung kann ein elektromagnetisch, pneumatisch oder hydraulisch betätigbares Mehrwegeventil sein. Das Mehrwegeventil kann aber auch nur von Hand betätigbar sein.

Die Steuereinheit ist vorzugsweise derart konfiguriert, dass in dem Betriebsmodus für eine Desinfektion des Hydrauliksystems die Ventileinrichtung in die Schaltstellung geschaltet ist, in der der Einlass mit mehreren Auslässen verbunden ist, so dass mehrere Strömungswege gleichzeitig mit heißer Flüssigkeit versorgt werden können.

Darüber hinaus sieht die Steuereinheit einen Betriebsmodus zur Durchführung der Blutbehandlung vor, wobei die Steuereinheit vorzugsweise derart konfiguriert ist, dass in dem Betriebsmodus für die Blutbehandlung die Ventileinrichtung in die Schaltstellung geschaltet ist, in der der Einlass nicht mit einem der Auslässe verbunden ist. Während der Blutbehandlung sind die Auslässe für die Zufuhr heißer Flüssigkeit zu den betreffenden Strömungswegen des Hydrauliksystems dann abgesperrt.

Eine besonders bevorzugte Ausführungsform der Ventileinrichtung weist einen hohlzylindrischen Gehäusekörper auf, in dem ein zylindrischer Ventilkörper drehbar angeordnet ist, wobei der Einlass und die Auslässe Bohrungen in dem Gehäusekörper sind und in dem Ventilkörper den Einlass mit den Auslässen verbindende Kanäle vorgesehen sind. Zum Drehen des Ventilkörpers ist vorzugsweise ein elektromagnetischer, insbesondere elektromotorischer, oder pneumatischer Stellantrieb vorgesehen. Die Ventileinrichtung kann aber beispielsweise auch einen in einem Gehäusekörper verschiebbaren Schieber aufweisen, wobei die Kanäle in dem Gehäusekörper ausgebildet sind.

Das erfindungsgemäße Verfahren zum Betreiben einer extrakorporalen Blutbehandlungsvorrichtung mit einem mehrere Strömungsstrecken umfassenden Hydrauliksystem, sieht vor, in einem Betriebsmodus für eine Heißdesinfektion des Hydrauliksystems eine auf eine vorgegebene Temperatur erwärmte Flüssigkeit, die an einer zentralen Stelle bereitgestellt wird, über Bypass-Leitungen von der zentralen Stelle mehreren Strömungsstrecken gleichzeitig zuzuführen. Eine der Strömungsstrecken, der die auf eine vorgegebene Temperatur erwärmte Flüssigkeit zugeführt wird, ist vorzugsweise eine zu einem Dialysator führende Strömungsstrecke. Die Strömungsstrecke kann auch eine zu einem Filter zur Erhöhung des Reinheitsgrades des Dialysats führende Strömungsstrecke sein. Für die Durchführung der Blutbehandlung kann die Flüssigkeitsströmung durch die Bypass-Leitungen unterbrochen werden.

Im Folgenden wird die Erfindung unter Bezugnahme auf die Zeichnungen im Einzelnen beschrieben.

Es zeigen:
- Fig. 1: die wesentlichen Komponenten eines Ausführungsbeispiels einer erfindungsgemäßen extrakorporalen Blutbehandlungsvorrichtung in stark vereinfachter schematischer Darstellung,
- Fig. 2: eine schematische Darstellung der Ventileinrichtung in einer ersten Schaltstellung,
- Fig. 3: eine schematische Darstellung der Ventileinrichtung in einer zweiten Schaltstellung,
- Fig. 4: eine schematische Darstellung der Ventileinrichtung in einer dritten Schaltstellung,
- Fig. 5: eine alternative Ausführungsform der erfindungsgemäßen extrakorporalen Blutbehandlungsvorrichtung und
- Fig. 6: eine weitere alternative Ausführungsform der erfindungsgemäßen extrakorporalen Blutbehandlungsvorrichtung.

Zunächst werden die wesentlichen Komponenten einer extrakorporalen Blutbehandlungsvorrichtung beschrieben.

Die Blutbehandlungsvorrichtung umfasst einen extrakorporalen Blutkreislauf I und ein Hydrauliksystem II. Das Hydrauliksystem umfasst mehrere Strömungswege, die wiederum, mehrere Leitungen aufweisen können. Nachfolgend werden nur einige der Strömungswege bzw. Leitungen beschrieben, die nur als Beispiele für die möglichen Strömungswege bzw. Leitungen einer Blutbehandlungsvorrichtung zu verstehen sind.

Der extrakorporale Blutkreislauf I schließt die Blutkammer 1 und das Hydrauliksystem II die Dialysatkammer 2 eines Dialysators 3 ein, der durch eine semipermeable Membran 4 in die Blutkammer 1 und Dialysatkammer 2 getrennt ist.

Zu der Blutkammer 1 des Dialysators 3 führt eine Blutzuführleitung 5, in die eine Blutpumpe 6 geschaltet ist, während von der Blutkammer 1 eine Blutabführleitung 7 abgeht. Die Blutzuführ- und -abführleitung 5, 7 bilden zusammen mit der Blutkammer 1 den extrakorporalen Blutkreislauf I der Blutbehandlungsvorrichtung.

Zur Bereitstellung des Dialysats weist das Hydrauliksystem B eine Dialysat-Aufbereitungseinrichtung 8 auf, die einen Einlass 8A für eine Flüssigkeit zur Herstellung des Dialysats, insbesondere Permeat (Reinwasser), und einen Auslass 8B aufweist. Das Permeat wird in einer Eingangskammer 9 gesammelt. Zur Herstellung des Dialysats wird das Permeat in einer Mischeinrichtung 10 mit einem oder mehreren Konzentraten gemischt.

Das Permeat kann der Blutbehandlungsvorrichtung an einem zentralen Anschluss 11 zugeführt werden, der über eine Zuflussleitung 12 mit dem Einlass der Eingangskammer 9 der Dialysat-Aufbereitungseinrichtung 8 verbunden ist. In der Zuflussleitung 12 ist ein Einlassventil 13 vorgesehen, so dass das Hydrauliksystem II abgetrennt werden kann.

Von dem Auslass 8B der Dialysat-Aufbereitungseinrichtung 8 führt eine Dialysatzuführleitung 14 zu dem Einlass 2A der Dialysatkammer 2 des Dialysators 3. Zum Anschluss an den Einlass 2A des Dialysators 3 weist die Dialysatzuführleitung 14 ein nur andeutungsweise dargestelltes Verbindungsstück 2AA auf, mit dem die Dialysatzuführleitung 14 mit dem Einlass des Dialysators 3 verbunden werden kann. Der Auslass 2B der Dialysatkammer 2 ist über eine Dialysatabführleitung 15 mit einem Ablauf 42 für gebrauchtes Dialysat verbunden. Zum Anschluss an den Auslass 2B des Dialysators 3 weist die Dialysatabführleitung 15 ein nur andeutungsweise dargestelltes Verbindungsstück 2BA auf, mit dem die Dialysatabführleitung 15 mit dem Auslass 2B des Dialysators 3 verbunden werden kann. Die Verbindungsstücke können sogenannte Hansenkupplungen sein.

Die Dialysatzuführleitung 14 weist einen ersten Abschnitt 14A auf, der von der Dialysat-Aufbereitungseinrichtung 8 zu der ersten Kammer 16A eines ersten Sterilfilters 16 führt. In den ersten Abschnitt 14A der Dialysatzuführleitung 14 ist die eine Kammer 17A einer Bilanziereinrichtung 17 geschaltet. Von der zweiten Kammer 16B des ersten Sterilfilters 16 geht der zweite Abschnitt 14B der Dialysatzuführleitung 14 ab, der zu der Dialysatkammer 3führt.

Die Dialysatabführleitung 15 teilt sich in zwei Abschnitte 15A, 15B und auf, die zu dem Ablauf 42 führen. In dem ersten Abschnitt 15A ist eine Dialysierflüssigkeitspumpe 18 geschaltet, während in dem zweiten Abschnitt 15B eine Ultrafiltratpumpe 19 geschaltet ist. In den ersten Abschnitt 15A ist darüber hinaus die andere Kammer 17B der Bilanziereinrichtung 17 geschaltet.

Für die Durchführung einer Heißdesinfektion des Hydrauliksystems II mit einer auf eine vorgegebene Temperatur erwärmten Flüssigkeit, die oberhalb von 80°C liegen sollte, verfügt die Blutbehandlungsvorrichtung über eine Heißdesinfektionseinrichtung 20, die eine Heizeinheit 20A zum Erhitzen der Flüssigkeit auf die erforderliche Temperatur aufweist. Als Flüssigkeit für die Heißdesinfektion findet die Flüssigkeit zur Herstellung des Dialysats Verwendung, insbesondere Permeat. Von der Eingangskammer 9 führt eine Leitung 21 zu dem Einlass der Heißdesinfektionseinrichtung 20. Der Auslass der Heißdesinfektionseinrichtung 20 ist über eine Leitung 22 mit dem Einlass einer Ventileinrichtung 23 verbunden, die nachfolgend noch im Einzelnen beschrieben wird.

Während der Heißdesinfektion ist der Dialysator 3 abgetrennt. Der zweite Abschnitt 14B der Dialysatzuführleitung 14 und der erste Abschnitt 15A der Dialysatabführleitung 15 sind während der Heißdesinfektion über eine Bypass-Leitung 43 miteinander verbunden. An der Bypass-Leitung 43 ist ein Bypassventil 44 vorgesehen, dass während der Blutbehandlung geschlossen und während der Heißdesinfektion geöffnet ist.

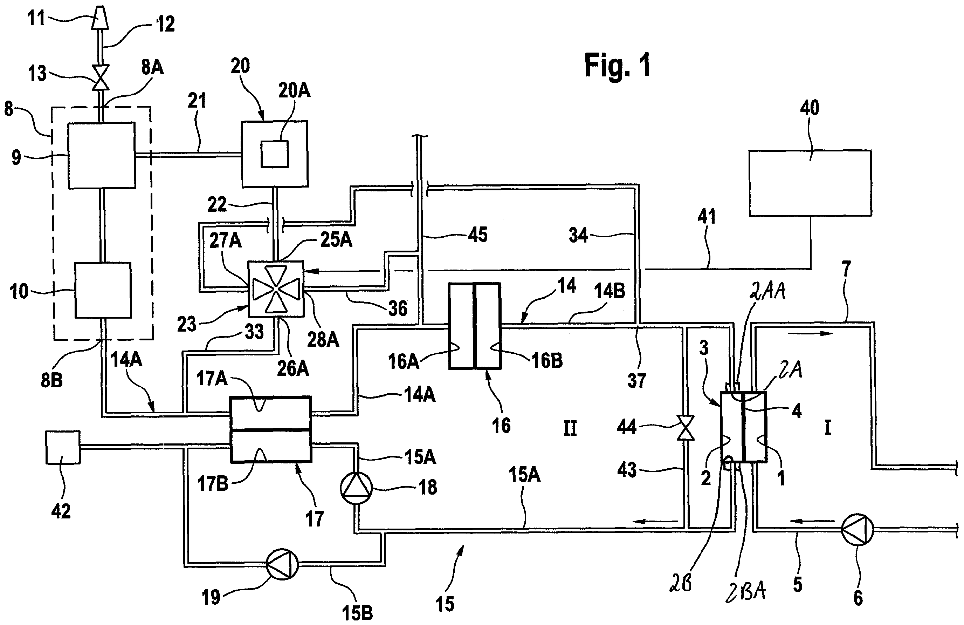

Die von der Heißdesinfektionseinrichtung 20 zu der Ventileinrichtung 23 führende Leitung 22 bildet eine Strömungsstrecke des Hydrauliksystems, über die erhitzte Flüssigkeit während der Heißdesinfektion dem Hydrauliksystem zugeführt wird. Eine weitere Strömungsstrecke bildet der zweite Abschnitt 14B der Dialysatzuführleitung 14, der zu dem Einlass 2A des Dialysators 3 führt, während der Heißdesinfektion aber von dem Dialysator abgetrennt ist. Diese Strömungstrecke ist als ein Beispiel für eine Strömungsstrecke zu verstehen, in der aufgrund relativ großer Leitungslängen die erhitzte Flüssigkeit unter die erforderliche Mindesttemperatur abgekühlt sein kann. Ein weiteres Beispiel für eine Strömungsstrecke bildet eine zu dem Sterilfilter 16 führende Leitung 45, von der in Fig. 1 nur ein Leitungsabschnitt dargestellt ist. Diese Strömungstrecke ist als ein Beispiel für eine Strömungsstrecke zu verstehen, die von der erhitzen Flüssigkeit nur schwer erreicht werden kann.

Darüber hinaus weist die Blutbehandlungsvorrichtung zur Ansteuerung der einzelnen Komponenten eine Steuereinheit 40 auf. Die Steuereinheit 40 sieht neben dem Betriebsmodus für die Durchführung der Blutbehandlung einen Betriebsmodus zur Durchführung der Heißdesinfektion vor. Während der Heißdesinfektion zirkuliert erhitztes Dialysat durch die Leitungen bzw. die mit den Leitungen in Fluidverbindung stehenden Komponenten des Hydrauliksystems.

Die Blutbehandlungsvorrichtung kann noch über weitere Leitungen, Absperrorgane und sowie sonstige Komponenten verfügen, die aber der Übersichtlichkeit halber nicht dargestellt sind.

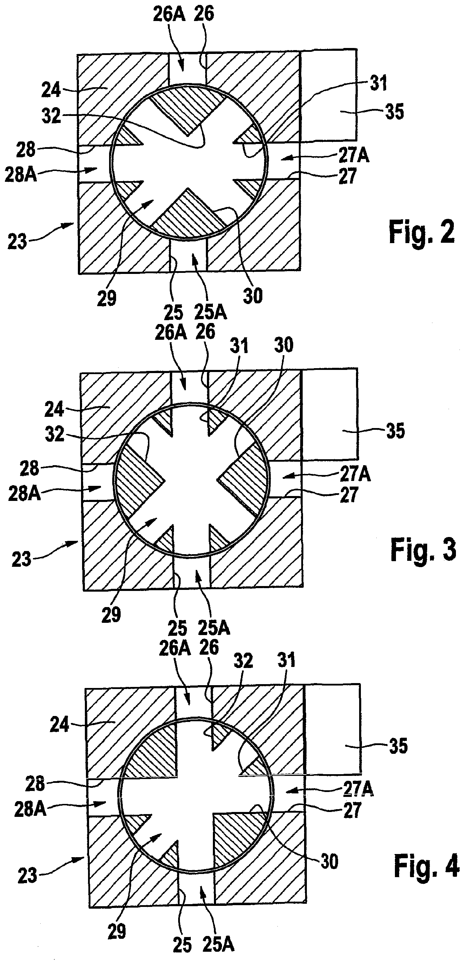

In den Figuren 2 bis 4 ist die Ventileinrichtung 23 in unterschiedlichen Schaltstellungen dargestellt. Die Ventileinrichtung 23 weist einen hohlzylindrischen Gehäusekörper 24 auf, der aus Kunststoff oder Metall bestehen kann. Der hohlzylindrische Gehäusekörper 24 weist vier Bohrungen 25, 26, 27, 28 auf, die sich von Außen in das Innere des Gehäusekörpers 24 erstrecken. In dem Gehäusekörper 24 ist ein zylindrischer Ventilkörper 29 drehbar gelagert, der ebenfalls aus Kunststoff oder Metall bestehen kann. Der Ventilkörper 29 weist drei Kanäle 30, 31, 32 auf, die sich in dessen Zentrum schneiden. Die Kanäle 30, 31, 32 in dem Ventilkörper 29 haben den gleichen Durchmesser wie die Bohrungen 25, 26, 27, 28 in dem Gehäusekörper 24. Eine der Bohrungen 25 bildet einen Einlass 25A und die anderen Bohrungen 26, 27, 28 bilden Auslässe 26A, 27A, 28A für die erhitze Flüssigkeit. Folglich hat die Ventileinrichtung einen Einlass 25A und drei Auslässe 26A, 27A, 28A.

Die Bohrungen 25, 26, 27, 28 sind an den Seiten des Gehäusekörpers 24 und die Kanäle 30, 31, 32 sind in dem Ventilkörper 29 derart ausgebildet, dass die Ventileinrichtung 23 die in den Figuren 2 bis 5 gezeigten Schaltstellungen einnehmen kann.

An den Einlass 25A der Ventileinrichtung 23 ist die von der Heißdesinfektionseinrichtung 20 abgehende Leitung 22 angeschlossen. Der erste Auslass 26A ist über eine Leitung 33 mit dem ersten Leitungsabschnitt 14A der Dialysatzuführleitung 14 stromauf der ersten Kammer 17A der Bilanziereinrichtung 17 verbunden. Der zweite Auslass 27A ist über eine Bypass-Leitung 34 mit dem zweiten Leitungsabschnitt 14B der Dialysatzuführleitung 14 stromab des Sterilfilters 16 verbunden, in der die Gefahr besteht, dass die erhitzte Flüssigkeit, wenn sie die anderen Strömungswege durchströmt hat, abgekühlt ist. Die Anschlussstelle 35 kann stromauf dem Anschluss der Bypass-Leitung 43 an den zweiten Abschnitt 14B der Dialysatzuführleitung 14 liegen. Der dritte Auslass 28A ist über eine Bypass-Leitung 36 mit der zu dem Sterilfilter 16 führenden Leitung 45 verbunden, die über die anderen Strömungswege nur schwer zu erreichen ist.

In der ersten Schaltstellung wird der Einlass 25A der Ventileinrichtung 23 von dem Ventilkörper 29 verschlossen. Der erste Auslass 26A, der dem Einlass 25A gegenüberliegt, wird ebenfalls von dem Ventilkörper 29 verschlossen. Der zweite und dritte Auslass 27A, 28A, die auf den anderen Seiten des Ventilkörpers 29 einander gegenüber liegen, sind hingegen über einen Kanal 31 in dem Ventilkörper 29 verbunden (Fig. 2).

In der zweiten Schaltstellung sind der Einlass 25A und der dem Einlass gegenüberliegende erste Auslass 26A über den Kanal 31 in dem Ventilkörper 29 verbunden, während der zweite und dritte Auslass 27A, 28A von dem Ventilkörper 29 verschlossen werden (Fig. 3).

In der dritten Schaltstellung sind der Einlass 25A und sämtliche Auslässe 26A, 27A, 28A über die Kanäle 30 und 32 in dem Ventilkörper 29 miteinander verbunden, so dass der Flüssigkeitsstrom der über den Einlass 25A zufließenden Flüssigkeit aufgeteilt wird (Fig. 4).

Die Ventileinrichtung 23 weist zum Verstellen des Ventilkörpers 29 in die einzelnen Schaltstellungen einen Stellantrieb 35 auf, der bei dem vorliegenden Ausführungsbeispiel ein elektromotorischer Stellantrieb ist. Der Stellantrieb 35 ist mit der Steuereinheit 40 der Blutbehandlungsvorrichtung über eine Steuerleitung 41 verbunden. Die Steuereinheit 40 ist derart konfiguriert, dass die in den Figuren gezeigten Schaltstellungen vorgegeben werden können.

In dem Betriebsmodus für die Durchführung der Blutbehandlung steuert die Steuereinheit 40 den Stellantrieb 35 der Ventileinrichtung 23 derart an, dass die Ventileinrichtung die erste Schaltstellung einnimmt, in der der Einlass 25A verschlossen ist, d.h. der Einlass nicht mit einem der Auslässe 26A, 27A, 28A verbunden ist. Folglich ist der für die Heißdesinfektion vorgesehen Teil des Hydrauliksystems II abgetrennt.

In dem Betriebsmodus für die Heißdesinfektion kann die Steuereinheit 40 den Stellantrieb 35 der Ventileinrichtung 23 derart ansteuern, dass die Ventileinrichtung die zweite Schaltstellung (Fig. 3) oder die dritte Schaltstellung (Fig. 4) einnimmt. Die zweite Schaltstellung entspricht einem konventionellen Desinfektionsbetrieb, bei dem die erhitzte Flüssigkeit, insbesondere Permeat, dem Hydrauliklsystem II nur an einer Stelle zugeführt wird. Das erhitzte Permeat strömt in den ersten Abschnitt 14A der Dialysatzuführleitung 14 stromauf der Bilanziereinrichtung 17. In der dritten Schaltstellung ist der Einlass 25A der Ventileinrichtung auch mit den beiden anderen Auslässen 27A, 28A verbunden, so dass erhitztes Permeat über die Bypass-Leitungen 34, 36 an zwei weiteren Stellen des Hydrauliksystem unter Umgehung nur schwer erreichbarer oder besonders langer Leitungsstrecken gleichzeitig zugeführt wird. Dadurch wird sichergestellt, dass das erhitzte Permeat in sämtlichen Leitungen des Hydrauliksystems die für die Heißdesinfektion erforderliche Mindesttemperatur hat.

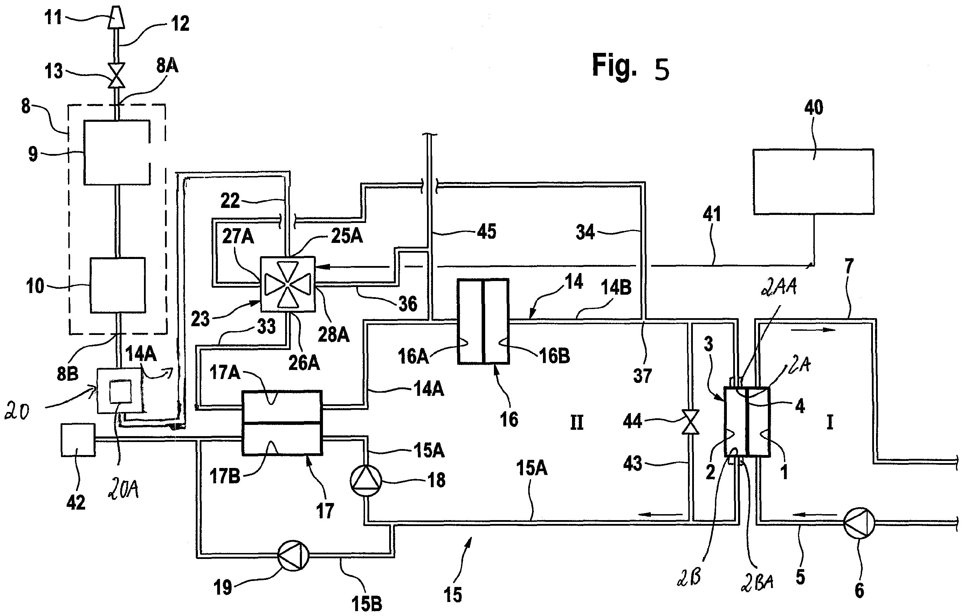

Fig. 5 zeigt weiteres Ausführungsbeispiel der Erfindung, das sich von der Ausführungsform von Fig. 1 nur durch die Anordnung der Heißdesinfektionseinrichtung 20 und der Ventileinrichtung 23 unterscheidet. Die einander entsprechenden Teile sind mit denselben Bezugszeichen versehen.

Bei dem Ausführungsbeispiel von Fig. 5 findet die Heizeinheit 20A der Heißdesinfektionseinrichtung 20 sowohl zum Erwärmen des Dialysats auf die erforderliche Temperatur für die Blutbehandlung als auch zum Erwärmen des Permeats auf die erforderliche Temperatur für die Heißdesinfektion Verwendung. Die Ventileinrichtung 23 ist bei der Ausführungsform von Fig. 5 in die Dialysatzuführleitung 14 geschaltet. Der Einlass 25A der Ventileinrichtung 23 ist mit einem zu der Ventileinrichtung 23 führenden Abschnitt der Dialysatzuführleitung 14 verbunden, während ein Auslass 26A der Ventileinrichtung 23 mit einem von der Ventileinrichtung 23 abgehenden Abschnitt der Dialysatzuführleitung 14 verbunden ist. Während der Blutbehandlung ist in einer Schaltstellung der Ventileinrichtung 23 der Einlass 25A der der Ventileinrichtung 23 mit dem Auslass 26 A der Ventileinrichtung 23 verbunden, wobei der Einlass 23 nicht mit den anderen Auslässen 27A und 28A verbunden ist. Für die Heißdesinfektion wird die Ventileinrichtung 23 in eine andere Schaltstellung geschaltet, in der der Einlass 25A der Ventileinrichtung 23 mit den beiden Auslässen 27A und 28A verbunden ist, so dass auf die für die Heißdesinfektion erforderliche Temperatur erwärmtes Permeat gleichzeitig über die zwei Strömungswege, die die Leitungen 34 bzw. 36 umfassen, dem Hydrauliksystem II direkt zugeführt wird. Dadurch werden lange Strömungswege vermieden, so dass sich das Permeat nicht abkühlen kann. Der Einlass 25A der Ventileinrichtung 23 kann in dieser Schaltstellung auch mit dem Auslass 26A verbunden sein, so dass erwärmtes Permeat über die Leitung 33 in die Bilanziereinrichtung 17 strömen kann.

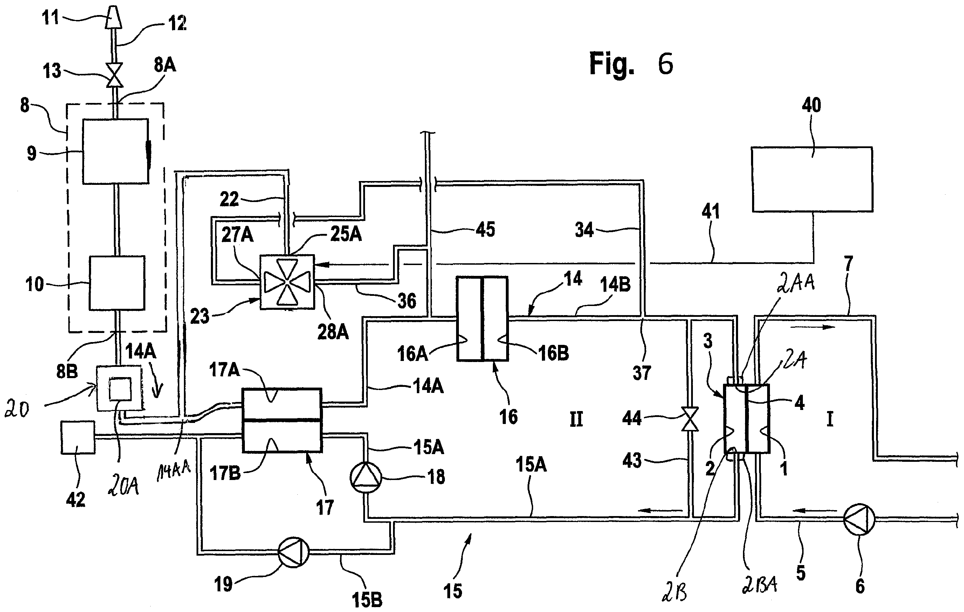

Fig. 6 zeigt weiteres Ausführungsbeispiel der Erfindung, das sich von der Ausführungsform von Fig. 5 nur durch die Anordnung und Ausbildung der Ventileinrichtung 23 unterscheidet. Die einander entsprechenden Teile sind wieder mit denselben Bezugszeichen versehen. Auch bei dem Ausführungsbeispiel von Fig. 6 findet die Heizeinheit 20A sowohl zum Erwärmen des Dialysats für die Blutbehandlung als auch zum Erwärmen des Permeats auf eine höhere Temperatur für die Heißdesinfektion Verwendung. Die Ventileinrichtung 23 ist aber nicht wie bei Fig. 5 in die Dialysatzuführleitung 14 geschaltet, sondern steht über eine Leitung 22 mit der Strömungsstrecke in Fluidverbindung ist, über die Flüssigkeit zur Herstellung von Dialysat bzw. Flüssigkeit für die Desinfektion des Hydrauliksystems II zuführbar ist. Die an der Anschlussstelle 14AA an die Dialysatzuführleitung 14 angeschlossene Leitung 22 verbindet einen Abschnitt der Dialysatzuführleitung 14, vorzugsweise einen Abschnitt zwischen der Heizeinheit 20A und der Bilanziereinrichtung 17, d.h. einen in enger räumlicher Nähe zur Heizeinheit 20A befindlichen Abschnitt, mit dem Einlass 25 A der Ventileinrichtung 23. Die Ventileinrichtung 23 hat bei dem vorliegenden Ausführungsbeispiel nur zwei Auslässe 27A und 28A, die mit den Leitungen 34 und 36 verbunden sind. Es können aber auch noch weitere Auslässe vorgesehen sein, die über weitere Strömungswege zu verschiedenen Punkten des Hydrauliksystems II führen.

## Patentansprüche

1. Extrakorporale Blutbehandlungsvorrichtung, die ein mehrere Strömungsstrecken umfassendes Hydrauliksystem (II) und eine Steuereinheit (40) aufweist, die einen Betriebsmodus für eine Desinfektion des Hydrauliksystems (II) mit einer Flüssigkeit vorsieht, die über eine der Strömungsstrecken zuführbar ist, wobei
das Hydrauliksystem (II) eine mehrere Schaltstellungen vorsehende Ventileinrichtung (23) aufweist, die einen Einlass (25A) für die Flüssigkeit zur Desinfektion des Hydrauliksystems und mehrere Auslässe (26A, 27A, 28A) aufweist, wobei der Einlass (25A) der Ventileinrichtung mit der Strömungsstrecke in Fluidverbindung ist, über die Flüssigkeit zur Desinfektion des Hydrauliksystems zuführbar ist, und die Auslässe (26A, 27A, 28A) der Ventileinrichtung jeweils mit einer anderen Strömungsstrecke des Hydrauliksystems in Fluidverbindung stehen,
in einer Schaltstellung der Ventileinrichtung (23) der Einlass (25A) mit mehreren Auslässen (26A, 27A, 28A) verbunden ist, und
das Hydrauliksystem (II) eine zu einem Dialysator (3) führende Dialysatzuführleitung (14) und eine von dem Dialysator abgehende Dialysatabführleitung (15) aufweist,
**dadurch gekennzeichnet, dass**
einer der Auslässe (26A, 27A, 28A) der Ventileinrichtung (23) über eine Bypass-Leitung (34) mit der Dialysatzuführleitung (14) verbunden ist, und/oder
das Hydrauliksystem (II) einen Filter (16) zur Erhöhung des Reinheitsgrades des Dialysats aufweist, der in einer der Strömungsstrecken des Hydrauliksystems angeordnet ist, wobei einer der Auslässe (26A, 27A, 28A) der Ventileinrichtung (23) über eine Bypass-Leitung (36) mit einer zu dem Filter (16) führenden Leitung (45) verbunden ist.

2. Extrakorporale Blutbehandlungsvorrichtung nach Anspruch 1, **dadurch gekennzeichnet, dass** in einer anderen Schaltstellung der Ventileinrichtung (23) der Einlass (25A) mit keinem der Auslässe (26A, 27A, 28A) verbunden ist.

3. Extrakorporale Blutbehandlungsvorrichtung nach Anspruch 1, **dadurch gekennzeichnet, dass** in einer anderen Schaltstellung der Ventileinrichtung (23) der Einlass (25A) mit einem der Auslässe (26A, 27A, 28A) verbunden ist.

4. Extrakorporale Blutbehandlungsvorrichtung nach einem der Ansprüche 1 bis 3, **dadurch gekennzeichnet, dass** die Blutbehandlungsvorrichtung eine Heißdesinfektionseinrichtung (20) mit einer Heizeinheit (20A) zum Erhitzen einer Flüssigkeit für eine Heißdesinfektion aufweist, wobei die Strömungsstrecke, über die Flüssigkeit zur Desinfektion des Hydrauliksystems zuführbar ist, mit der Heißdesinfektionseinrichtung (20) in Fluidverbindung steht.

5. Extrakorporale Blutbehandlungsvorrichtung nach einem der Ansprüche 1 bis 4, **dadurch gekennzeichnet, dass** die Blutbehandlungsvorrichtung einen Anschluss (11) für die Zufuhr einer Flüssigkeit aufweist, mit dem die Strömungsstrecke, über die die Flüssigkeit zur Desinfektion des Hydrauliksystems (II) zuführbar ist, in Fluidverbindung steht.

6. Extrakorporale Blutbehandlungsvorrichtung nach einem der Ansprüche 1 bis 5, **dadurch gekennzeichnet, dass** das Hydrauliksystem (II) eine zu einem Dialysator (3) führende Dialysatzuführleitung (14) und eine von dem Dialysator abgehende Dialysatabführleitung (15) aufweist, wobei der Einlass (25A) der Ventileinrichtung (23) mit der zu dem Dialysator (3) führenden Dialysatzuführleitung (14) in Fluidverbindung steht.

7. Extrakorporale Blutbehandlungsvorrichtung nach Anspruch 6, **dadurch gekennzeichnet, dass** der Dialysator (3) einen Einlass (2A) zum Zuführen von Dialysat und einen Auslass (2B) zum Abführen von Dialysat aufweist, wobei die Dialysatzuführleitung (14) ein Anschlussstück (2AA) zum Anschluss an den Einlass (2A) des Dialysators (3) und die Dialysatabführleitung (15) ein Anschlussstück (2BA) zum Anschluss an den Auslass (2B) des Dialysators (3) aufweist, so dass der Dialysator für die Desinfektion von dem Hydrauliksystem (II) abtrennbar ist.

8. Extrakorporale Blutbehandlungsvorrichtung nach Anspruch 7, **dadurch gekennzeichnet, dass** Ventilanordnung in die Dialysatzuführleitung (14) geschaltet ist.

9. Extrakorporale Blutbehandlungsvorrichtung nach einem der Ansprüche 1 bis 8, **dadurch gekennzeichnet, dass** die Ventileinrichtung (23) derart ausgebildet ist, dass die Ventileinrichtung von der Steuereinheit (40) betätigbar ist.

10. Extrakorporale Blutbehandlungsvorrichtung nach einem der Ansprüche 1 bis 9, **dadurch gekennzeichnet, dass** die Steuereinheit (40) derart konfiguriert ist, dass in dem Betriebsmodus für eine Desinfektion des Hydrauliksystems (II) die Ventileinrichtung (23) in die Schaltstellung geschaltet ist, in der der Einlass (25A) mit mehreren Auslässen (26A, 27A, 28A) verbunden ist.

11. Extrakorporale Blutbehandlungsvorrichtung nach einem der Ansprüche 1 bis 10, **dadurch gekennzeichnet, dass** die Steuereinheit (40) einen Betriebsmodus zur Durchführung der Blutbehandlung vorsieht, wobei die Steuereinheit (40) derart konfiguriert ist, dass in dem Betriebsmodus für die Blutbehandlung die Ventileinrichtung (23) in die Schaltstellung geschaltet ist, in der der Einlass (25A) mit keinem der Auslässe (26A, 27A, 28A) verbunden ist.

12. Extrakorporale Blutbehandlungsvorrichtung nach einem der Ansprüche 1 bis 11, **dadurch gekennzeichnet, dass** die Ventileinrichtung (23) einen hohlzylindrischen Gehäusekörper (24) aufweist, in dem ein zylindrischer Ventilkörper (29) drehbar angeordnet ist, wobei der Einlass (25A) und die Auslässe (26A, 27A, 28A) Bohrungen (25; 26, 27, 28) in dem Gehäusekörper (24) sind und in dem Ventilkörper (29) den Einlass (25A) mit den Auslässen (26A, 27A, 28A) verbindende Kanäle (30, 31, 32) vorgesehen sind.

13. Extrakorporale Blutbehandlungsvorrichtung nach Anspruch 12, **dadurch gekennzeichnet, dass** zum Drehen des Ventilkörpers (29) ein elektromagnetischer, insbesondere elektromotorischer, oder pneumatischer Stellantrieb (35) vorgesehen ist.

14. Verfahren zum Betreiben einer extrakorporalen Blutbehandlungsvorrichtung mit einem mehrere Strömungsstrecken umfassenden Hydrauliksystem, wobei in einem Betriebsmodus für eine Heißdesinfektion des Hydrauliksystems eine auf eine vorgegebene Temperatur erwärmte Flüssigkeit, die an einer zentralen Stelle bereitgestellt wird, von der zentralen Stelle über Bypass-Leitungen (34, 36) mehreren Strömungsstrecken gleichzeitig zugeführt wird,
**dadurch gekennzeichnet, dass**
eine der Strömungsstrecken, der die auf eine vorgegebene Temperatur erwärmte Flüssigkeit über eine Bypass-Leitung (34) zugeführt wird, eine zu einem Dialysator (3) führende Strömungsstrecke ist, und/oder
eine der Strömungsstrecken, der die auf eine vorgegebene Temperatur erwärmte Flüssigkeit über eine Bypass-Leitung (36) zugeführt wird, eine zu einem Filter (16) zur Erhöhung des Reinheitsgrades des Dialysats führende Strömungsstrecke ist.

15. Verfahren nach Anspruch 14, **dadurch gekennzeichnet, dass** in einem Betriebsmodus für die Durchführung der Blutbehandlung die Flüssigkeitsströmung durch die Bypass-Leitungen (34, 36) unterbrochen wird.

## Claims

1. Extracorporeal blood treatment device comprising a hydraulic system (II) comprising a plurality of flow paths, the blood treatment device comprising a control unit (40) providing an operating mode for disinfecting the hydraulic system (II) with a liquid that can be supplied via one of the flow paths,
the hydraulic system (II) comprising a multi-switch valve device (23) having an inlet (25A) for the liquid for disinfecting the hydraulic system and a plurality of outlets (26A, 27A, 28A), the inlet (25A) of the valve device being in fluid communication with the flow path, via which liquid for disinfecting the hydraulic system can be supplied, and the outlets (26A, 27A, 28A) of the valve device being each in fluid communication with another flow path of the hydraulic system,
in a switching position of the valve device (23) the inlet (25A) being connected to a plurality of outlets (26A, 27A, 28A), and
the hydraulic system (II) having a dialysate supply conduit (14) leading to a dialyzer (3) and a dialysate discharge conduit (15) leading away from the dialyzer,
**characterized in that**
one of the outlets (26A, 27A, 28A) of the valve device (23) is connected via a bypass conduit (34) to the dialysate supply conduit (14), and/or
the hydraulic system (II) comprises a filter (16) for increasing the degree of purity of the dialysate, which is arranged inside one of the flow paths of the hydraulic system, wherein one of the outlets (26A, 27A, 28A) of the valve device (23) is connected via a bypass conduit (36) to a conduit (45) leading to the filter (16).

2. Extracorporeal blood treatment device according to claim 1, **characterized in that** in another switching position of the valve device (23), the inlet (25A) is connected to none of the outlets (26A, 27A, 28A).

3. Extracorporeal blood treatment device according to claim 1, **characterised in that** in another switching position of the valve device (23), the inlet (25A) is connected to one of the outlets (26A, 27A, 28A).

4. Extracorporeal blood treatment device according to one of claims 1 to 3, **characterised in that** the blood treatment device comprises a hot disinfection device (20) with a heating unit (20A) for heating a liquid for hot disinfection, wherein the flow path through which the liquid for disinfecting the hydraulic system can be supplied, is in fluid communication with the hot disinfection device (20).

5. Extracorporeal blood treatment device according to one of claims 1 to 4, **characterized in that** the blood treatment device has a connection (11) for the supply of a liquid, with which connection the flow path, via which the liquid for disinfecting the hydraulic system (II) can be supplied, is in fluid communication.

6. Extracorporeal blood treatment device according to one of claims 1 to 5, **characterized in that** the hydraulic system (II) has a dialysate supply conduit (14) leading to a dialyzer (3) and a dialysate discharge conduit (15) leading away from the dialyzer, wherein the inlet (25A) of the valve device (23) is in fluid communication with the dialysate supply conduit (14) leading to the dialyzer (3).

7. Extracorporeal blood treatment device according to claim 6, **characterized in that** the dialyzer (3) has an inlet (2A) for supplying dialysate and an outlet (2B) for discharging dialysate, wherein the dialysate supply conduit (14) has a connector (2AA) for connecting to the inlet (2A) of the dialyzer (3) and the dialysate discharge conduit (15) has a connector (2BA) for connecting to the outlet (2B) of the dialyzer (3), so that the dialyzer is separable from the hydraulic system (II) for disinfection.

8. Extracorporeal blood treatment device according to claim 7, **characterized in that** the valve arrangement is switched inside the dialysate supply conduit (14).

9. Extracorporeal blood treatment device according to one of claims 1 to 8, **characterized in that** the valve device (23) is designed such that the valve device can be actuated by the control unit (40).

10. Extracorporeal blood treatment device according to one of claims 1 to 9, **characterized in that** the control unit (40) is configured such that in the operating mode for disinfecting the hydraulic system (II) the valve device (23) is switched to the switching position in which the inlet (25A) is connected to a plurality of outlets (26A, 27A, 28A).

11. Extracorporeal blood treatment device according to one of claims 1 to 10, **characterized in that** the control unit (40) provides an operating mode for performing blood treatment, wherein the control unit (40) is configured such that in the operating mode for blood treatment, the valve device (23) is switched to the switching position, in which the inlet (25A) is not connected to any of the outlets (26A, 27A, 28A).

12. Extracorporeal blood treatment device according to one of claims 1 to 11, **characterized in that** the valve device (23) comprises a hollow cylindrical housing body (24) in which a cylindrical valve body (29) is rotatably arranged, wherein the inlet (25A) and the outlets (26A, 27A, 28A) are bores (25; 26, 27, 28) in the housing body (24), and in the valve body (29) passages (30, 31, 32) connecting the inlet (25A) to the outlets (26A, 27A, 28A) are provided.

13. Extracorporeal blood treatment device according to claim 12, **characterised in that** for rotating the valve body (29) an electromagnetic, in particular electromotive, or pneumatic actuator (35) is provided.

14. Method for operating an extracorporeal blood treatment device having a hydraulic system (II) which comprises several flow paths, **characterized in that** in an operating mode for a hot disinfection of the hydraulic system, a liquid heated to a predetermined temperature provided at a central point is simultaneously supplied from the central point via bypass conduits (34, 36) to a plurality of flow paths,
**characterized in that** one of the flow paths, which is supplied with the liquid heated to a predetermined temperature via a bypass conduit (34), is a flow path leading to a dialyzer (3), and/or
one of the flow paths, which is supplied with a liquid heated to a predetermined temperature via a bypass conduit (36) is a flow path leading to a filter (16) for increasing the degree of purity of the dialysate.

15. Method according to claim 14, **characterised in that** in an operating mode, for carrying out the blood treatment, the liquid flow passing through the bypass conduits (34, 36) is interrupted.

## Revendications

1. Dispositif de traitement extracorporel du sang, qui présente un système hydraulique (II) comprenant plusieurs tronçons d'écoulement et une unité de commande (40), qui prévoit un mode de fonctionnement pour une désinfection du système hydraulique (II) avec un liquide, qui est apte à être amené par l'intermédiaire d'un des tronçons d'écoulement, dans lequel
le système hydraulique (II) présente un équipement de soupape (23) prévoyant plusieurs positions de commutation, qui présente une entrée (25A) pour le liquide pour la désinfection du système hydraulique et plusieurs sorties (26A, 27A, 28A), dans lequel l'entrée (25A) de l'équipement de soupape est en communication fluidique avec le tronçon d'écoulement, par l'intermédiaire duquel est apte à être amené le liquide pour la désinfection du système hydraulique, et les sorties (26A, 27A, 28A) de l'équipement de soupape sont en communication fluidique respectivement avec un autre tronçon d'écoulement du système hydraulique, l'entrée (25A) est reliée à plusieurs sorties (26A, 27A, 28A) dans une position de commutation de l'équipement de soupape (23), et
le système hydraulique (II) présente un conduit d'amenée de dialysat (14) menant à un dialyseur (3) et un conduit d'évacuation de dialysat (15) partant du dialyseur, **caractérisé en ce que**
l'une des sorties (26A, 27A, 28A) de l'équipement de soupape (23) est reliée au conduit d'amenée de dialysat (14) par l'intermédiaire d'un conduit de dérivation (34), et/ou
le système hydraulique (II) présente un filtre (16) pour augmenter le degré de pureté du dialysat, qui est disposé dans l'un des tronçons d'écoulement du système hydraulique, dans lequel une des sorties (26A, 27A, 28A) de l'équipement de soupape (23) est reliée à un conduit (45) menant au filtre (16) par l'intermédiaire d'un conduit de dérivation (36).

2. Dispositif de traitement extracorporel du sang selon la revendication 1 , **caractérisé en ce que** dans une autre position de commutation de l'équipement de soupape (23), l'entrée (25A) n'est reliée à aucune des sorties (26A, 27A, 28A).

3. Dispositif de traitement extracorporel du sang selon la revendication 1, **caractérisé en ce que** dans une autre position de commutation de l'équipement de soupape (23), l'entrée (25A) est reliée à une des sorties (26A, 27A, 28A).

4. Dispositif de traitement extracorporel du sang selon l'une quelconque des revendications 1 à 3, **caractérisé en ce que** le dispositif de traitement du sang présente un équipement de désinfection à chaud (20) avec une unité de chauffage (20A) pour chauffer un liquide pour une désinfection à chaud, dans lequel le tronçon d'écoulement, par l'intermédiaire duquel est apte à être amené le liquide pour la désinfection du système hydraulique, est en communication fluidique avec l'équipement de désinfection à chaud (20).

5. Dispositif de traitement extracorporel du sang selon l'une quelconque des revendications 1 à 4, **caractérisé en ce que** le dispositif de traitement du sang présente un raccordement (11) pour l'amenée d'un liquide, avec lequel le tronçon d'écoulement, par l'intermédiaire duquel est apte à être amené le liquide pour la désinfection du système hydraulique (II), est en communication fluidique.

6. Dispositif de traitement extracorporel du sang selon l'une quelconque des revendications 1 à 5, **caractérisé en ce que** le système hydraulique (II) présente un conduit d'amenée de dialysat (14) menant vers un dialyseur (3) et un conduit d'évacuation de dialysat (15) partant du dialyseur, dans lequel l'entrée (25A) de l'équipement de soupape (23) est en communication fluidique avec le conduit d'amenée de dialysat (14) menant au dialyseur (3).

7. Dispositif de traitement extracorporel du sang selon la revendication 6, **caractérisé en ce que** le dialyseur (3) présente une entrée (2A) pour amener du dialysat et une sortie (2B) pour évacuer du dialysat, dans lequel le conduit d'amenée de dialysat (14) présente une pièce de raccordement (2AA) destinée à être raccordée à l'entrée (2A) du dialyseur (3) et le conduit d'évacuation de dialysat (15) présente une pièce de raccordement (2BA) destinée à être raccordée à la sortie (2B) du dialyseur (3) de sorte que le dialyseur soit séparable du système hydraulique (II) pour la désinfection.

8. Dispositif de traitement extracorporel du sang selon la revendication 7, **caractérisé en ce que** l'ensemble de soupape est branché dans le conduit d'amenée de dialysat (14).

9. Dispositif de traitement extracorporel du sang selon l'une quelconque des revendications 1 à 8, **caractérisé en ce que** l'équipement de soupape (23) est réalisé de telle manière que l'équipement de soupape soit actionnable par l'unité de commande (40).

10. Dispositif de traitement extracorporel du sang selon l'une quelconque des revendications 1 à 9, **caractérisé en ce que** l'unité de commande (40) est configurée de telle manière que dans le mode de fonctionnement pour une désinfection du système hydraulique (II), l'équipement de soupape (23) est branché dans la position de commutation, dans laquelle l'entrée (25A) est reliée à plusieurs sorties (26A, 27A, 28A).

11. Dispositif de traitement extracorporel du sang selon l'une quelconque des revendications 1 à 10, **caractérisé en ce que** l'unité de commande (40) prévoit un mode de fonctionnement pour mettre en œuvre le traitement du sang, dans lequel l'unité de commande (40) est configurée de telle manière que dans le mode de fonctionnement pour le traitement du sang, l'équipement de soupape (23) est branché dans la position de commutation, dans laquelle l'entrée (25A) n'est reliée à aucune des sorties (26A, 27A, 28A).

12. Dispositif de traitement extracorporel du sang selon l'une quelconque des revendications 1 à 11, **caractérisé en ce que** l'équipement de soupape (23) présente un corps de boîtier (24) cylindrique creux, dans lequel est disposé de manière à pouvoir tourner un corps de soupape (29) cylindrique, dans lequel l'entrée (25A) et les sorties (26A, 27A, 28A) sont des alésages (25 ; 26, 27, 28) dans le corps de boîtier (24) et des canaux (30, 31, 32) reliant l'entrée (25A) aux sorties (26A, 27A, 28A) sont prévus dans le corps de soupape (29).

13. Dispositif de traitement extracorporel du sang selon la revendication 12, **caractérisé en ce qu'**un entraînement de réglage (35) électromagnétique, en particulier électromotorisé ou pneumatique est prévu pour faire tourner le corps de soupape (29).

14. Procédé pour faire fonctionner un dispositif de traitement extracorporel du sang avec un système hydraulique comprenant plusieurs tronçons d'écoulement, dans lequel dans un mode de fonctionnement pour une désinfection à chaud du système hydraulique, un liquide chauffé à une température prédéfinie, qui est fourni sur un emplacement central, est amené de manière simultanée à plusieurs tronçons d'écoulement depuis l'emplacement central par l'intermédiaire de conduits de dérivation (34, 36),
**caractérisé en ce que**
l'un des tronçons d'écoulement, auquel le liquide chauffé à une température prédéfinie est amené par l'intermédiaire d'un conduit de dérivation (34), est un tronçon d'écoulement menant vers un dialyseur (3), et/ou
l'un des tronçons d'écoulement, auquel le liquide chauffé à une température prédéfinie est amené par l'intermédiaire d'un conduit de dérivation (36), est un tronçon d'écoulement menant à un filtre (16) pour augmenter le degré de pureté du dialysat.

15. Procédé selon la revendication 14, **caractérisé en ce que** dans un mode de fonctionnement pour la mise en œuvre du traitement du sang, l'écoulement de liquide est interrompu par les conduits de dérivation (34, 36).
